(19) **Europäisches Patentamt European Patent Office Office européen des brevets**

(11) **EP 4 031 688 B1**

(12) **EUROPEAN PATENT SPECIFICATION**

(45) Date of publication and mention of the grant of the patent:
**12.07.2023 Bulletin 2023/28**

(21) Application number: **20801170.0**

(22) Date of filing: **21.10.2020**

(51) International Patent Classification (IPC):
**C12Q 1/6886** (2018.01)   **G16B 20/00** (2019.01)
**G16B 40/20** (2019.01)

(52) Cooperative Patent Classification (CPC):
**C12Q 1/6886; G16B 20/00; G16B 40/20;**
C12Q 2600/112; C12Q 2600/156

(86) International application number:
**PCT/EP2020/079612**

(87) International publication number:
**WO 2021/078794 (29.04.2021 Gazette 2021/17)**

(54) **IN VITRO METHOD FOR DETERMINING THE RISK OF DEVELOPING BREAST CANCER IN A SUBJECT**

IN-VITRO-VERFAHREN ZUR BESTIMMUNG DES RISIKOS DER ENTWICKLUNG VON BRUSTKREBS BEI EINER PERSON

PROCÉDÉ IN VITRO POUR DÉTERMINER LE RISQUE DE DÉVELOPPEMENT DU CANCER DU SEIN CHEZ UN SUJET

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.10.2019 EP 19382918**

(43) Date of publication of application:
**27.07.2022 Bulletin 2022/30**

(73) Proprietor: **Sistemas Genómicos, S.L.**
**46980 Paterna (Valencia) (ES)**

(72) Inventors:
• **BENÍTEZ ORTIZ, Javier**
  **28029 Madrid (ES)**
• **MIÑAMBRES HERRAIZ, Rebeca**
  **46980 Valencia (ES)**
• **TRIVIÑO PARDO, Juan Carlos**
  **46980 Valencia (ES)**
• **ROSA FERRERRO, Ricardo**
  **46980 Valencia (ES)**
• **RIBAS DESPUIG, Gloria**
  **46980 Valencia (ES)**
• **CEBA PERIS, Andrea**
  **46980 Valencia (ES)**
• **RUBIO SOLSONA, Estrella**
  **46980 Valencia (ES)**

• **LEGARDA CRISTÓBAL, Garikoitz**
  **46980 Valencia (ES)**
• **DÍAZ CHACÓN, José Leonardo**
  **46980 Valencia (ES)**
• **CORTINA GIL, Belén**
  **46980 Valencia (ES)**
• **BERNAD PALOMARES, Lucía**
  **46980 Valencia (ES)**
• **CABO DÍEZ, Miguel**
  **46980 Valencia (ES)**
• **GONZÁLEZ NEIRA, Ana Isabel**
  **28029 Madrid (ES)**
• **PITA MACPHERSON, Guillermo**
  **28029 Madrid (ES)**

(74) Representative: **Manuel Illescas y Asociados, S.L.**
**C/ Príncipe de Vergara 197, Oficina 1°A**
**28002 Madrid (ES)**

(56) References cited:
**KR-A- 20140 098 695**

- SHIEH YIWEY ET AL: "Breast cancer risk prediction using a clinical risk model and polygenic risk score", BREAST CANCER RESEARCH AND TREATMENT, SPRINGER , NY, US, vol. 159, no. 3, 26 August 2016 (2016-08-26), pages 513-525, XP036054433, ISSN: 0167-6806, DOI: 10.1007/S10549-016-3953-2 [retrieved on 2016-08-26]
- MINA LIDA A ET AL: "Polygenic Risk Scores in Breast Cancer", CURRENT BREAST CANCER REPORTS, SPRINGER US, BOSTON, vol. 11, no. 3, 13 August 2019 (2019-08-13), pages 117-122, XP036874970, ISSN: 1943-4588, DOI: 10.1007/S12609-019-00320-8 [retrieved on 2019-08-13]
- HATEF DARABI ET AL: "Breast cancer risk prediction and individualised screening based on common genetic variation and breast density measurement", BREAST CANCER RESEARCH, CURRENT MEDICINE GROUP LTD, GB, vol. 14, no. 1, 7 February 2012 (2012-02-07), page R25, XP021119757, ISSN: 1465-5411, DOI: 10.1186/BCR3110
- CHARMAINE PEI LING LEE ET AL: "Mammographic Breast Density and Common Genetic Variants in Breast Cancer Risk Prediction", PLOS ONE, vol. 10, no. 9, 24 September 2015 (2015-09-24), pages 1-16, XP055592050, DOI: 10.1371/journal.pone.0136650
- JENNA LILYQUIST ET AL: "Common Genetic Variation and Breast Cancer Risk-Past, Present, and Future", CANCER EPIDEMIOLOGY, BIOMARKERS AND PREVENTION., vol. 27, no. 4, 30 January 2018 (2018-01-30), pages 380-394, XP055687987, US ISSN: 1055-9965, DOI: 10.1158/1055-9965.EPI-17-1144

**Description**

**TECHNICAL FIELD**

The present invention relates to *in vitro* methods for determining the risk of developing breast cancer, particularly in women. In particular, the present invention relates to methods in which genotypic risks related to single nucleotide polymorphisms (SNPs), risks related to phenotypic characteristics and family history and risks related to interactions between variables associated with said phenotypic characteristics are combined.

**BACKGROUND ART**

Breast cancer is the type of cancer that mostly affects women, both in developed and developing countries. The annual incidence and mortality represent, worldwide, 1,677,000 new cases of breast cancer and 522,000 associated deaths. According to the latest epidemiological data published by EUCAN for the EU-5 member countries (Germany, Spain, France, Italy and the United Kingdom), the incidence varied between 84.9 and 129.2 cases per 100,000 inhabitants, while mortality varied between 16.7 and 24.8 per 100,000 inhabitants (in 2012). The 5-year prevalence of diagnosis for these countries was between 104,210 and 279,045 patients in 2012.

Being able to diagnose breast cancer in the early stages of the disease is very important since an early diagnosis positively influences the survival of patients. The 3-year survival of patients diagnosed in stage I is found to be over 99%, while for patients diagnosed in stage IV drops to 27%. Several studies have shown that the implementation of breast screening can reduce breast cancer mortality by around 20%-35% in women of 50 years of age or older. However, the current screening programs implemented in clinical routine for several decades present a series of limitations due to the application of general guidelines for action without taking into account the individualised risk profile of each woman.

In recent years, genome-wide association studies (GWAS) have been used intensively to identify genetic variants associated with the risk of breast cancer.

The "Breast Cancer Association Consortium" (BCAC) consortium was founded in 2004, aimed at understanding the architecture of different types of common cancer, which was the starting point for the implementation of the projects COGs (study of more than 40,000 women with cancer and 40,000 controls), Oncoarray (study of 600,000 genetic markers in 275,000 women) and other GWAS studies a few years later (Evans et al.). Following these multicentre studies in the Caucasian population based on massive genotyping, the main risk factors associated with the development of breast cancer were identified. Currently, the breast cancer association describes the following factors: family history, mutations in target genes (BRCA) and genotypic risk factors of genetic susceptibility (single nucleotide polymorphisms, SNPs (Michailidou et al. 2013; Michailidou et al. 2015; Michailidou et al. 2017; Nickels et al.) and phenotypic risk factors (breast density, age at menopause and age at first child, etc.) (Pollán et al.). A strategy capable of integrating all these factors efficiently could improve the prevention of individual risk of developing the disease.

There are several models for predicting the risk of breast cancer throughout a woman's life, each based on a series of risk factors. Because the factors considered are different, individual risk may also vary between models. For example, EP2438193A1 describes methods and systems to assess the overall risk of developing breast cancer and describes performing a clinical risk assessment using the models of Gail, Claus, BOADICEA, Jonker, the Extended Formula of Claus, Tyrer-Cuzick and the Manchester Scoring System and combining said clinical risk assessment with the assessment of genetic risks based on the presence of certain SNPs. Also the publication by Shieh Yiwey et al. 2016, discloses brest cancer prediction using a combination of a clinical risk model and polygenic risk score.

However, the probability calculation models described in the state of the art consider a series of very limited risk factors.

**SUMMARY OF THE INVENTION**

In this document, "single nucleotide polymorphisms" (SNP) refer to changes of a single pair of nitrogenous bases of the genome. Typically, a single nucleotide polymorphism is the replacement of a nucleotide with another nucleotide at the polymorphic site. A "polymorphic site" is the locus wherein a variation occurs. The deletion of an individual nucleotide, or the insertion of an individual nucleotide, also results in single nucleotide polymorphisms. The term "polymorphism" refers to the appearance of two or more alternative genomic sequences or alleles between different genomes or individuals.

In this document, "odds ratio" (OR) refers to the likelihood of an event occurring in a study group, compared to the likelihood of the same event occurring in a control group. In the risk of breast cancer, the value of the OR ratio is usually used to determine if being exposed to a certain substance or factor increases the risk of cancer. The results are interpreted as follows:

- OR = 1: Both groups have the same chances of exposure, so exposure probably does not increase the risk of cancer.
- OR > 1: Exposure may increase the risk of cancer.
- OR < 1: Exposure can reduce the risk of cancer.

In this document, "logistic regression" refers to a regression method that estimates the probability of a qualitative binary variable depending on a quantitative variable. One of the main applications of logistic regression is that of binary classification, wherein the observations are classified into one group or the other depending on the value taken by the variable used as a predictor.

In this document, the "Cox model", also called "proportional hazards model" refers to a regression model used for survival analysis. The Cox model uses the hazard function and detects existing relationships between the risk that occurs in a given individual in the study at a certain time and according to some independent and/or explanatory variables. The Cox model enables evaluation of which variables within a set of variables have a relation or influence on the risk function and therefore also on the survival function, since both functions are connected.

In this document, the "Z-score" refers to a measure of how many standard deviations below or above the population mean is a given data. Values observed above the population mean have positive Z-score scores, while values below the population mean have negative Z-score scores.

In this document, the "receiver operating characteristic curve" (ROC curve) refers to a graphical representation of sensitivity versus specificity for a binary classifier system. Another interpretation of this curve is the representation of the true positive rate (TPR) versus the false positive rate (FPR). Points above the diagonal line represent good classification results (better than a random classification). Classification results above the diagonal are better the further they are from the diagonal.

The technical problem to be solved is to provide an improved method of determining the risk of developing breast cancer, in relation to greater precision in the sense of greater sensitivity and specificity, with respect to the methods described in the state of the art, which combine genotypic risk and phenotypic risk to obtain the risk of developing breast cancer in a subject.

The method of the invention, as characterised in the claims, provides a solution to said technical problem.

The invention provides an improved method for determining the risk of developing breast cancer, with greater precision, in the sense of greater sensitivity and specificity, with respect to the methods described in the state of the art, which combine genotypic risk and phenotypic risk to obtain the risk of developing breast cancer in a subject.

The method of the invention to determine the risk of developing breast cancer integrates risk information associated with family history, genotypic risk and phenotypic risk, said combination of information resulting in an individual risk value for a subject of suffering from breast cancer. This enables the general population to be stratified into different risk groups and thus selecting the subjects that are at higher risk of developing breast cancer in order to provide a more thorough clinical follow-up.

Thus, the present invention provides an *in vitro* method for determining the risk of developing breast cancer in a subject, wherein said method comprises combining the following parameters in a model:

- the polygenic risk score (PRS) calculated from the risks associated with the presence of single nucleotide polymorphisms (SNPs) in a biological sample obtained from said subject,
- the risk associated in said subject to the factors of breast density, biopsy, age at menopause, age, family history and age at first child and
- the risk associated in said subject to the age-breast density and age- age at menopause interactions, wherein said interactions are interactions obtained by regression,

wherein said risk of developing breast cancer in said subject is calculated from a model based on the combination of said parameters and wherein the result obtained in said model is indicative of said risk of developing breast cancer in said subject, wherein said single nucleotide polymorphisms (SNPs) are rs10069690, rs10088218, rs1045485, rs10472076, rs10474352, rs10771399, rs10816625, rs10931936, rs10941679, rs10965163, rs10995190, rs10995201, rs11196174, rs11196175, rs11199914, rs11249433, rs113577745, rs11552449, rs11571833, rs11814448, rs11820646, rs11977670, rs12422552, rs12443621, rs12710696, rs1292011, rs13039229, rs13066793, rs13281615, rs13294895, rs13329835, rs13365225, rs13387042, rs1353747, rs1432679, rs1466785, rs1550623, rs16857609, rs16886113, rs16991615, rs17356907, rs17631303, rs17817449, rs183211, rs184577, rs1895062, rs204247, rs2046210, rs2236007, rs2253407, rs2290203, rs2290854, rs2304277, rs2363956, rs2420946, rs2588809, rs2736108, rs2747652, rs27633, rs2823093, rs2943559, rs2981579, rs2992756, rs344008, rs3757322, rs3760982, rs3803662, rs3903072, rs4442975, rs45631563, rs4691139, rs4733664, rs4784227, rs4808801, rs4849887, rs4973768, rs554219, rs58058861, rs58847541, rs6001930, rs614367, rs616488, rs619373, rs6472903, rs6504950, rs6507583, rs6682208, rs67397200, rs6762644, rs6815814, rs6828523, rs704010, rs7072776, rs720475, rs72749841, rs72755295, rs72826962, rs7297051, rs73161324, rs7529522, rs75915166, rs78269692, rs8009944, rs8170, rs865686, rs889312, rs909116, rs9303542, rs9348512, rs9397437, rs941764, rs9693444, rs9790517, rs9790879, rs9833888, rs527616 and rs2380205,
wherein the combination, in said model, of the PRS score, the risk associated with the factors and the risk associated with the interactions, is carried out by the formula

$$BRM = \beta_0 + \beta_1 PRS + \beta_2 OR_{DM} + \beta_3 OR_{BIOP} + \beta_4 OR_{E1} + \beta_5 OR_{AF} + \beta_6 OR_{EM} + \beta_7 E + \beta_8 E + \beta_9 E$$

wherein $OR_{DM}$ is the value of the OR associated with breast density, $OR_{BIOP}$ is the value of the OR associated with the biopsy, $OR_{E1}$ is the value of the OR associated with the age at first child, $OR_{AF}$ is the value of the OR associated with the family history, $OR_{EM}$ is the value of the OR associated with the age at menopause, E is the age, $\beta_0$, $\beta_1$, $\beta_2$, $\beta_3$, $\beta_4$, $\beta s$, $\beta_6$, $\beta_7$, $\beta_8$ and $\beta_9$ are coefficients determined in said model with a training cohort, $\beta_1$ is the coefficient associated with the PRS, $\beta_2$ is the coefficient associated with the breast density, $\beta_3$ is the coefficient associated with the biopsy, $\beta_4$ is the coefficient associated with the age at first child, $\beta_5$ is the coefficient associated with the family history, $\beta_6$ is the coefficient associated with the age at menopause, $\beta_7$ is the coefficient associated with the age, $\beta_8$ is the coefficient associated with the age-breast density interaction and $\beta_9$ is the coefficient associated with the age-age at menopause interaction,
wherein said model is a Cox proportional hazards model, wherein said risk of developing breast cancer in a subject is calculated by the formula

$$h(t) = h_o(t) exp \left( \sum_{i=1}^{m} \beta_i V_i \right)$$

where h(t) is the risk function of breast cancer, t is the time, $h_0(t)$ is the baseline hazard function, m is the number of risk factors, $V_i$ are the risk factors: polygenic risk score (PRS), breast density, biopsy, age at menopause, age, family history, age at first child, age-breast density interaction and age-age at menopause interaction; and $\beta_i$ corresponds to the coefficients $\beta_0$-$\beta_9$.

In this method of the invention for determining the risk of developing breast cancer, said age-breast density and age- age at menopause interactions obtained by regression indicate that said risk of developing breast cancer in said subject, associated with breast density and the age at menopause is conditioned by age.

In a preferred embodiment of the method of the invention for determining the risk of developing breast cancer, said subject is a woman.

**Polygenic Risk Score (PRS)**

In the present invention, the value of PRS for each woman is calculated using the formula:

$$\prod_{k=1}^{n} OR_k^{x_k}$$

where each SNP is identified by a *k* value, where *k* is a value between 1 and n, where n is the total number of SNPs, $OR_k$ is the value of the OR (odds ratio) associated with SNP k, where $x_k$ is the number of alleles for SNP k and $x_k$ can have a value selected from the group consisting of 0, 1 and 2.

Therefore, in a preferred embodiment of the method of the invention for determining the risk of developing breast cancer, the PRS score is calculated by the formula

$$\prod_{k=1}^{n} OR_k^{x_k}$$

where each SNP is identified by a k value, where k is a value between 1 and n, where n is the total number of SNPs, $OR_k$ is the value of the OR (odds ratio) associated with SNP k, where $x_k$ is the number of alleles for SNP k and $x_k$ can have a value selected from the group consisting of 0, 1 and 2.

In the method of the invention for determining the risk of developing breast cancer, 117 single nucleotide polymorphisms (SNPs) shown in Table 1 and described in the scientific publications indicated in said Table are used in the estimation of the Polygenic Risk Score, PRS.

It has been described how the presence of each variant is associated with a certain risk (Ghoussaini et al., Couch et al.; Michailidou et al. 2017; Gaudet et al., Osorio et al., Bojesen et al.).

The OR values for each SNP are indicated in Table 1. In Table 1, the total number of SNPs is 117 and, therefore, n = 117.

Table 1. SNPs used in genetic analysis

| k | SNP | $OR_k$ | Reference |
|---|---|---|---|
| 1 | rs10069690 | 1.06 | 1 |
| 2 | rs10088218 | 1 | 2 |
| 3 | rs1045485 | 0.88 | 1 |
| 4 | rs10472076 | 1.03 | 1 |
| 5 | rs10474352 | 0.94 | 3 |
| 6 | rs10771399 | 0.79 | 1 |
| 7 | rs10816625 | 1.11 | 3 |
| 8 | rs10931936 | 0.88 | 1 |
| 9 | rs10941679 | 1.15 | 1 |
| 10 | rs10965163 | 1 | 4 |
| 11 | rs10995190 | 0.86 | 1 |
| 12 | rs10995201 | 0.9 | 3 |
| 13 | rs11196174 | 1 | 2 |
| 14 | rs11196175 | 1 | 2 |
| 15 | rs11199914 | 0.96 | 1 |
| 16 | rs11249433 | 1.11 | 1 |
| 17 | rs113577745 | 1.08 | 3 |
| 18 | rs11552449 | 1.04 | 1 |
| 19 | rs11571833 | 1.35 | 1 |
| 20 | rs11814448 | 1.12 | 1 |
| 21 | rs11820646 | 0.96 | 1 |
| 22 | rs11977670 | 1.06 | 3 |
| 23 | rs12422552 | 1.06 | 1 |
| 24 | rs12443621 | 1.11 | 1 |
| 25 | rs12710696 | 1.03 | 1 |
| 26 | rs1292011 | 0.92 | 1 |

| k | SNP | OR$_k$ | Reference |
|---|---|---|---|
| 27 | rs13039229 | 1 | 4 |
| 28 | rs13066793 | 0.94 | 3 |
| 29 | rs13281615 | 1.11 | 1 |
| 30 | rs13294895 | 1.06 | 3 |
| 31 | rs13329835 | 1.07 | 1 |
| 32 | rs13365225 | 0.91 | 3 |
| 33 | rs13387042 | 1.2 | 1 |
| 34 | rs1353747 | 0.96 | 1 |
| 35 | rs1432679 | 1.08 | 1 |
| 36 | rs1466785 | 1 | 5 |
| 37 | rs1550623 | 0.94 | 1 |
| 38 | rs16857609 | 1.06 | 1 |
| 39 | rs16886113 | 1 | 4 |
| 40 | rs16991615 | 1.1 | 3 |
| 41 | rs17356907 | 0.91 | 1 |
| 42 | rs17631303 | 1 | 2 |
| 43 | rs17817449 | 0.95 | 1 |
| 44 | rs183211 | 1 | 2 |
| 45 | rs184577 | 1 | 4 |
| 46 | rs1895062 | 0.94 | 3 |
| 47 | rs204247 | 1.04 | 1 |
| 48 | rs2046210 | 1.11 | 1 |
| 49 | rs2236007 | 0.93 | 1 |
| 50 | rs2253407 | 1 | 4 |
| 51 | rs2290203 | 0.94 | 3 |
| 52 | rs2290854 | 1 | 2 |
| 53 | rs2304277 | 1 | 5 |
| 54 | rs2363956 | 1.19 | 1 |
| 55 | rs2420946 | 1 | 4 |
| 56 | rs2588809 | 1.06 | 1 |
| 57 | rs2736108 | 1 | 6 |
| 58 | rs2747652 | 0.94 | 3 |
| 59 | rs27633 | 1 | 4 |
| 60 | rs2823093 | 0.94 | 1 |
| 61 | rs2943559 | 1.1 | 1 |
| 62 | rs2981579 | 1.43 | 1 |
| 63 | rs2992756 | 1.06 | 3 |
| 64 | rs344008 | 1 | 2 |

(continued)

| k | SNP | OR$_k$ | Reference |
|---|---|---|---|
| 65 | rs3757322 | 1.08 | 3 |
| 66 | rs3760982 | 1.05 | 1 |
| 67 | rs3803662 | 1.2 | 1 |
| 68 | rs3903072 | 0.97 | 1 |
| 69 | rs4442975 | 0.89 | 3 |
| 70 | rs45631563 | 0.81 | 3 |
| 71 | rs4691139 | 1 | 2 |
| 72 | rs4733664 | 1 | 4 |
| 73 | rs4784227 | 1.23 | 3 |
| 74 | rs4808801 | 0.93 | 1 |
| 75 | rs4849887 | 0.91 | 1 |
| 76 | rs4973768 | 1.11 | 1 |
| 77 | rs554219 | 1.21 | 3 |
| 78 | rs58058861 | 1.06 | 3 |
| 79 | rs58847541 | 1.08 | 3 |
| 80 | rs6001930 | 1.12 | 1 |
| 81 | rs614367 | 1.15 | 1 |
| 82 | rs616488 | 0.94 | 1 |
| 83 | rs619373 | 1 | 4 |
| 84 | rs6472903 | 0.94 | 1 |
| 85 | rs6504950 | 0.95 | 1 |
| 86 | rs6507583 | 0.92 | 3 |
| 87 | rs6682208 | 1 | 2 |
| 88 | rs67397200 | 1 | 2 |
| 89 | rs6762644 | 1.05 | 1 |
| 90 | rs6815814 | 1.06 | 3 |
| 91 | rs6828523 | 0.91 | 1 |
| 92 | rs704010 | 1.07 | 1 |
| 93 | rs7072776 | 1.05 | 1 |
| 94 | rs720475 | 0.96 | 1 |
| 95 | rs72749841 | 0.93 | 3 |
| 96 | rs72755295 | 1.15 | 3 |
| 97 | rs72826962 | 1.2 | 3 |
| 98 | rs7297051 | 0.89 | 3 |
| 99 | rs73161324 | 1.06 | 3 |
| 100 | rs7529522 | 1.06 | 3 |
| 101 | rs75915166 | 1.28 | 3 |
| 102 | rs78269692 | 1.09 | 3 |

(continued)

| k | SNP | $OR_k$ | Reference |
|---|---|---|---|
| 103 | rs8009944 | 0.88 | 1 |
| 104 | rs8170 | 1.26 | 1 |
| 105 | rs865686 | 0.89 | 1 |
| 106 | rs889312 | 1.13 | 1 |
| 107 | rs909116 | 1.17 | 1 |
| 108 | rs9303542 | 1 | 2 |
| 109 | rs9348512 | 1 | 4 |
| 110 | rs9397437 | 1.17 | 3 |
| 111 | rs941764 | 1.03 | 1 |
| 112 | rs9693444 | 1.06 | 1 |
| 113 | rs9790517 | 1.04 | 1 |
| 114 | rs9790879 | 1.1 | 1 |
| 115 | rs9833888 | 1.06 | 3 |
| 116 | rs527616 | 0.97 | 1 |
| 117 | rs2380205 | 0.94 | 1 |
| Reference 1: Ghoussaini et al.; reference 2: Couch et al.; reference 3: Michailidou et al. 2017; reference 4: Gaudet et al.; reference 5: Osorio et al.; reference 6: Bojesen et al. | | | |

In the method of the invention for determining the risk of developing breast cancer, the risk of developing breast cancer is calculated from the BRM (Brecan Risk Model) formula, a global interaction model, which is shown below:

$$BRM = \beta_0 + \beta_1 PRS + \beta_2 OR_{DM} + \beta_3 OR_{BIOP} + \beta_4 OR_{E1} + \beta_5 OR_{AF}$$
$$+ \beta_6 OR_{EM} + \beta_7 E + \beta_8 E + \beta_9 E$$

where $OR_{DM}$ is the value of the OR associated with breast density, $OR_{BIOP}$ is the value of the OR associated with the biopsy, $OR_{E1}$ is the value of the OR associated with the age at first child, $OR_{AF}$ is the value of the OR associated with family history, $OR_{EM}$ is the value of the OR associated with the age at menopause, E is the age, $\beta_0$, $\beta_1$, $\beta_2$, $\beta_3$, $\beta_4$, $\beta_5$, $\beta_6$, $\beta_7$, $\beta_8$ and $\beta_9$ are coefficients determined in said model with a training cohort, $\beta_1$ is the coefficient associated with the PRS, $\beta_2$ is the coefficient associated with the breast density, $\beta_3$ is the coefficient associated with the biopsy, $\beta_4$ is the coefficient associated with the age at first child, $\beta_8$ is the coefficient associated with the family background, $\beta_6$ is the coefficient associated with the age at menopause, $\beta_7$ is the coefficient associated with the age, $\beta_8$ is the coefficient associated with the age-breast density interaction and $\beta_9$ is the coefficient associated with the age- age at menopause interaction.

The BRM formula includes the factors that have the greatest statistical significance (see Table 8) in the case-control study with the training cohort (see Example 1): PRS genotypic risk, breast density (DM), biopsy (BIOP), age at menopause (MS), age (E), age-density breast interaction and age- age at menopause interaction, as well as other factors that have lower statistical significance (family history (AF) and age at first child (E1)) in said Example 1. In the survival analysis of Example 2, all factors have statistical significance.

The OR values and the Polygenic Risk Score (PRS) are multiplied by the weights or coefficients $\beta_1$, $\beta_2$, $\beta_3$, $\beta_4$, $\beta_5$, $\beta_6$, $\beta_7$, $\beta_8$, and $\beta_9$ that were obtained from a logistic regression in the case-control study with the training cohort. Weights provide each of the genotypic/phenotypic variables with greater or lesser importance in the calculation of total risk. These weights depend on the training cohort in which the model is trained.

In the BRM formula, the influence of age has been included, not so much as an individual factor, but its influence with the interaction with both breast density and with age at menopause categories, since it has been observed that both interactions have an important statistical significance, and therefore their inclusion in the algorithm implies an improvement in the results obtained.

Therefore, in the method of the invention for determining the risk of developing breast cancer, in said model of the PRS score, the risks associated with the factors and the risks associated with the interactions are combined as indicated in the formula

$$BRM = \beta_0 + \beta_1 PRS + \beta_2 OR_{DM} + \beta_3 OR_{BIOP} + \beta_4 OR_{E1} + \beta_5 OR_{AF} + \beta_6 OR_{EM} + \beta_7 E + \beta_8 E + \beta_9 E$$

where $OR_{DM}$ is the value of the OR associated with breast density, $OR_{BIOP}$ is the value of the OR associated with the biopsy, $OR_{E1}$ is the value of the OR associated with the age at first child, $OR_{AF}$ is the value of the OR associated with the family history, $OR_{EM}$ is the value of the OR associated with the age at menopause, E is the age, $\beta_0$, $\beta_1$, $\beta_2$, $\beta_3$, $\beta_4$, $\beta_5$, $\beta_6$, $\beta_7$, $\beta_8$ and $\beta_9$ are coefficients determined in said model with a training cohort, $\beta_1$ is the coefficient associated with the PRS, $\beta_2$ is the coefficient associated with the breast density, $\beta_3$ is the coefficient associated with the biopsy, $\beta_4$ is the coefficient associated with the age at first child, $\beta_8$ is the coefficient associated with the family history, $\beta_6$ is the coefficient associated with the age at menopause, $\beta_7$ is the coefficient associated with the age, $\beta_8$ is the coefficient associated with the age-breast density interaction and $\beta_9$ is the coefficient associated with the age- age at menopause interaction.

In a preferred embodiment of the method of the invention for determining the risk of developing breast cancer, $\beta_0$ has a value in any of the following ranges: $0.01\text{-}100\cdot10^{22}$, $0.1\text{-}10\cdot10^{22}$, $0.1\text{-}4\cdot10^{22}$, $0.1\text{-}2\cdot10^{22}$ and $0.5\text{-}1.5\cdot10^{22}$.

In a more preferred embodiment of the method of the invention for determining the risk of developing breast cancer, the coefficients $\beta_1$-$\beta_9$ have values between 0 and 2.

In a more preferred embodiment of the method of the invention for determining the risk of developing breast cancer, $\beta_1$ has a value in any of the following ranges: 0-1, 0-0.8, 0-0.6, 0.1-0.4 and 0.2-0.3. In a more preferred embodiment of the method of the invention, $\beta_2$ has a value in any of the following ranges: 0-1, 0-0.8, 0-0.6, 0-0.4, 0-0.2 and 0.05-0.15. In a more preferred embodiment of the method of the invention, $\beta_3$ has a value in any of the following ranges: 0-1, 0-0.8, 0-0.7, 0-0.5, 0-0.3 and 0.1-0.2. In a more preferred embodiment of the method of the invention, $\beta_4$ has a value in any of the following ranges: 0-1, 0-0.8, 0.2-0.7, 0.3-0.6 and 0.4-0.5. In a more preferred embodiment of the method of the invention, $\beta_5$ has a value in any of the following ranges: 0.5-1.5, 0.7-1.2, 0.8-1.1 and 0.9-1. In a more preferred embodiment of the method of the invention, $\beta_6$ has a value in any of the following ranges: 0-1, 0-0.5, 0-0.3, 0-0.2 and 0-0.1. In a more preferred embodiment of the method of the invention, $\beta_7$ has a value in any of the following ranges: 0-1, 0-0.8, 0.2-0.7, 0.3-0.6 and 0.4-0.5. In a more preferred embodiment of the method of the invention, $\beta_8$ has a value in any of the following ranges: 0.5-1.5, 0.8-1.3, 0.9-1.2 and 1-1.1. In a more preferred embodiment of the method of the invention, $\beta_9$ has a value in any of the following ranges: 1-2, 1.4-1.9, 1.5-1.8 and 1.6-1.7.

In another preferred embodiment of the method of the invention for determining the risk of developing breast cancer, said regression is logistic regression.

In the method of the invention for determining the risk of developing breast cancer, said model is a Cox proportional hazards model, to predict the risk of developing breast cancer in said subject, wherein said risk is calculated by the formula

$$h(t) = h_o(t) exp\left(\sum_{i=1}^{m} \beta_i V_i\right)$$

where h(t) is the risk function of breast cancer, t is the time, $h_0(t)$ is the baseline hazard function, m is the number of risk factors, $V_i$ are the risk factors, polygenic risk score (PRS), breast density, biopsy, age at menopause, age, family history, age at first child, age-breast density interaction and age- age at menopause interaction; and $\beta_i$ corresponds to the coefficients described above.

Additionally, the present invention also provides a method for stratifying or classifying subjects according to the risk of developing breast cancer, which comprises determining the risk of developing breast cancer in said subjects according to the method of the invention described above and assigning or classifying subjects into low risk, populational risk and high risk groups.

In a preferred embodiment of said stratification method of the invention, said subject is a woman.

In a preferred embodiment of said stratification method of the invention, a subject is assigned:

- in the low risk group if the BRM value is equal to or greater than 0 and less than or equal to 0.5,

- in the populational risk group when the BRM value is greater than 0.5 and less than 2,
- in the high risk group when the BRM value is equal to or greater than 2.

The present invention also provides a method for selecting subjects at risk of developing breast cancer which comprises determining the risk of developing breast cancer of said subjects according to the method of the invention described above and selecting subjects based on the value of said risk of developing breast cancer.

In a preferred embodiment of said selection method of the invention, said subject is a woman.

In a preferred embodiment of said selection method of the invention, subjects assigned or classified into a high risk group are selected when the BRM value is equal to or greater than 2.

**Phenotypic risks**

Family history

Table 2. OR values used in the method of the invention for determining the risk of developing breast cancer, associated with the phenotypic characteristic family history for a woman of age $\geq$ 50 years.

|  |  | OR |
|---|---|---|
| No family antecent |  | 1 |
| One family antecedent | 1st degree: $\geq$ 50 | 1.7 |
|  | 1st degree: < 50 | 1.8 |
|  | 2nd degree | 1.6 |
| Two family antecedents | 1st degree: | 1.6 |
|  | 2nd degree | 1.6 |
| Three or more family antecedents |  | 1.6 |

Table 3. OR values used in the method of the invention for determining the risk of developing breast cancer, associated with the phenotypic characteristic family history for a woman of age < 50 years.

|  |  | OR |
|---|---|---|
| No family antecedent |  | 1 |
| One family antecedent | 1st degree: $\geq$ 50 | 1.8 |
|  | 1st degree: < 50 | 3.3 |
|  | 2nd degree | 1.7 |
| Two family antecedents | 1st degree: | 1.7 |
|  | 2nd degree | 1.7 |
| Three or more family antecedents |  | 1.7 |

The values in Tables 2 and 3 are based on the data described in Pharoah et al.

Biopsy

Table 4. OR values used in the method of the invention to determine the risk of developing breast cancer, associated with the phenotypic characteristic biopsy. The values in this table are described in Pollán et al.

| Characteristic | No. of controls (%) | No. of breast cancer cases (%) | OR (95% CI) | P-value |
|---|---|---|---|---|
| Previous biopsy | | | | <0.001 |
| Yes | 4281 (91.3) | 1001 (85.4) | 1.00 (reference) | |
| No | 407 (8.7) | 171 (146) | 1.82 (1.50-2.22) | |

Breast density

Table 5. OR values used in the method of the invention to determine the risk of developing breast cancer, associated with the phenotypic characteristic breast density. The values in this table are described in Pollán et al.

| Characteristic | No. of controls (%) | No. of breast cancer cases (%) | OR (95% CI) | P-value |
|---|---|---|---|---|
| Mammographic Density (%) | | | | <0.001 |
| 0-10 | 1711 (36.5) | 263 (22.4) | 1.00 (reference) | |
| 11-25 | 1136 (24.3) | 244 (20.8) | 1.52 (1.25-1.85) | |
| 26-50 | 1158 (24.8) | 375 (32.0) | 2.47 (2.05-2.98) | |
| 51-75 | 529 (11.3) | 215 (18.3) | 3.27 (2.62-4.09) | |
| >75 | 135 (2.9) | 59 (5.0) | 3.74 (2.63-5.32) | |

Age at Menopause

Table 6. OR values used in the method of the invention to determine the risk of developing breast cancer, associated with the phenotypic characteristic age at menopause. The values in this table are described in Pollán et al.

| Characteristic | No. of controls (%) | No. of breast cancer cases (%) | OR (95% CI) | P-value |
|---|---|---|---|---|
| Age at Menopause (years) | | | | 0.001 |
| ≤45 | 777 (16.6) | 147 (12.5) | 1.00 (reference) | |
| 46-50 | 1244 (26.5) | 298 (25.4) | 1.25 (1.01-1.56) | |
| >50 | 1035 (22.1) | 193 (25.0) | 1.48 (1.17-1.87) | |
| Premenopausal | 1631 (34.8) | 434 (37.0) | 1.44 (1.14-1.83) | |

Age at first child

Table 7. OR values used in the method of the invention for determining the risk of developing breast cancer, associated with the phenotypic characteristic age at first child. The values in this table are described in Pollán et al.

| Characteristic | No. of controls (%) | No. of breast cancer cases (%) | OR (95% CI) | P-value |
|---|---|---|---|---|
| Age at first child (years) | | | | <0.001 |

(continued)

| Characteristic | No. of controls (%) | No. of breast cancer cases (%) | OR (95% CI) | P-value |
|---|---|---|---|---|
| ≤20 | 102 (2.2) | 13(1.1) | 0.51 (0.28-0.91) | |
| 20-24 | 1441 (30.7) | 293 (25.0) | 0.81 (0.68-0.95) | |
| 25-29 | 1823 (38.9) | 457 (39.0) | 1.00 (reference) | |
| 30-34 | 522 (11.1) | 134 (11.4) | 1.03 (0.83-1.28) | |
| ≥35 | 175 (3.7) | 57 (4.9) | 1.31 (0.95-1.80) | |
| Childless | 625 (13.3) | 218 (18.6) | 1.41 (1.17-1.70) | |

**BRIEF DESCRIPTION OF THE FIGURES**

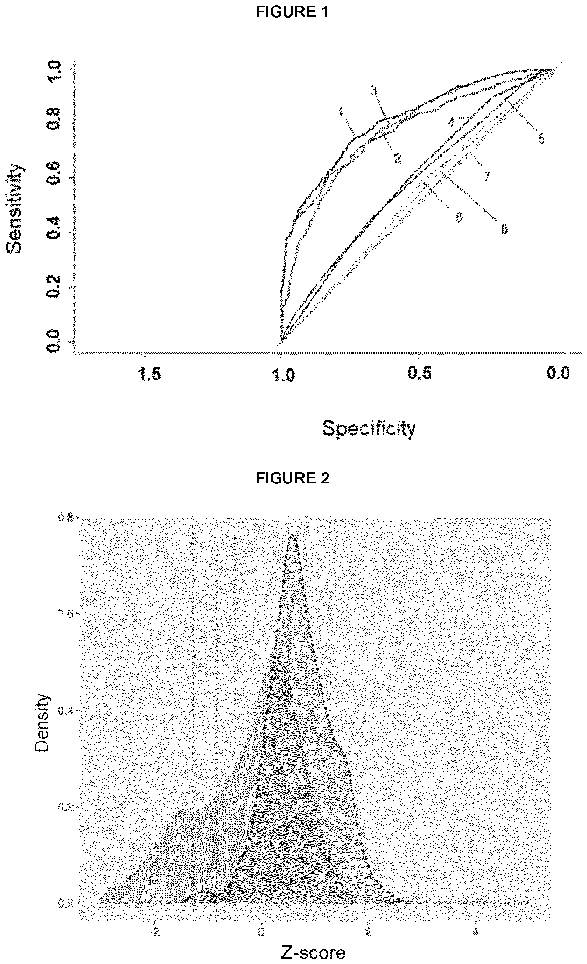

Figure 1. ROC curves obtained with different models with the training cohort. Curve 1 is the ROC curve obtained with the BRM model (a global interaction model). Curve 2 is the ROC curve obtained with a model that uses the risk associated with the factors PRS genotypic risk, breast density, biopsy, age at menopause, age, age at first child and family history (i.e., the model from curve 2 differs from the BRM model in that it does not use the age-breast density and age- age at menopause interactions). ROC curves obtained with models that use only the risk associated with a single factor are also represented: breast density (curve 3), PRS genotypic risk (curve 5), age at first child (curve 6), biopsy (curve 7) and family history (curve 8). Curve 3 represents the ROC curve obtained with a model that uses the phenotypic factors breast density, biopsy, age at menopause, age and age at first child.

Figure 2. Z-score graph of the training cohort, representing cases (continuous line with dots) and controls (continuous line).

Figure 3. OR value distribution between cases (continuous line with dots) and controls (continuous line).

Figure 4. Percentage of controls and cases in each risk category (high risk, populational and low risk). Cases are represented by black bars and controls by white bars.

Figure 5. Survival curves with the Cox model associated, individually, to breast density for an OR value associated with breast density of 1 (curve 1), 1.52 (curve 2), 2.47 (curve 3), 3.27 (curve 4) and 3.74 (curve 5).

Figure 6. Survival curves with the Cox model associated, individually, at age at first child for an OR value associated with the age at first child of 0.51 (curve 1), 0.81 (curve 2), 1 (curve 3), 1.03 (curve 4), 1.31 (curve 5) and 1.41 (curve 6).

Figure 7. Survival curves with the Cox model associated, individually, to the age at menopause for an OR value associated with the age at menopause of 1 (curve 1), 1.25 (curve 2), 1.44 (curve 3) and 1.48 (curve 4).

Figure 8. Survival curves with the Cox model associated, individually, to the family history for an OR value associated with the family history of 1 (curve 1), 1.6 (curve 2), 1.7 (curve 3), 1.8 (curve 4) and 3.3 (curve 5).

Figure 9. Survival curves with the Cox model associated, individually, to the genotypic risk determined by the PRS polygenic risk score for an OR value associated with the PRS of 0.4200859 (curve 1), 0.5554001 (curve 2), 0.676850842 (curve 3), 0.6907142 (curve 4), 0.8260284 (curve 5), 0.9613425 (curve 6), 1.0966567 (curve 7), 1.2319709 (curve 8), 1.367285 (curve 9), 1.5025992 (curve 10), 1.64779095 (curve 11), 1.8607595 (curve 12), 2.536816107 (curve 13), 2.814491683 (curve 14) and 2.94314836 (curve 15).

Figure 10. Inferred survival curves with the Cox model for a first woman. The solid line represents the mean value and the dashed lines represent the lower and upper values with a confidence interval of the 95%.

Figure 11. Inferred survival curves with the Cox model for a second woman. The solid line represents the mean value and the dashed lines represent the lower and upper values with a confidence interval of the 95%.

Figure 12. Inferred survival curves with the Cox model for a first woman (curve 1) and for a second woman (curve 2). The solid line represents the mean value and the dashed lines represent the lower and upper values with a confidence interval of the 95%.

## DESCRIPTION OF EMBODIMENTS

### Methods

All women filled out a questionnaire including possible family history. Informed consent was obtained from all women.

A blood sample was obtained from all women, and their DNA was automatically extracted using a DNA extractor (MagNA Pure, Roche, Mannheim, Germany), then the DNA was quantified by the Picogreen fluorimetric technique and finally genotyped using a Openarray custom genotyping array containing the SNPs associated with breast cancer in Table 1.

### Example 1. Case-control study with the training cohort

The working cohort was called the training cohort. The analysis was focused on case-control studies. The training cohort had 1095 women from the Caucasian population, of which 462 were cases and 633 controls. The median age in the training cohort was 52 years and the age ranged between 20 and 83 years.

Healthy women were called controls. Controls were used to analyse the individual risk of developing breast cancer in order to identify the risk group to which they belonged (low, moderate, high).

The women of the training cohort who at that time had breast cancer and women who at that time did not have breast cancer but were subsequently positively diagnosed within a period of 1 to 10 years were considered as cases.

The values of the phenotypic factors of women who initially did not have breast cancer, but were subsequently diagnosed, were available at the time of their entry into the program, when they did not have breast cancer (2008 or 2013 respectively). The values of these factors were not available at the time of carrying out the method of the invention.

Cases were used to analyse the concordance between the actual incidence and the risk of developing breast cancer determined by the method of the invention.

### Logistic regression in the BRM model

Logistic regression was performed in the case-control study with the training cohort and the results obtained are shown in Table 8. The value obtained for $\beta_0$ was $1.06316 \cdot 10^{22}$.

As indicated above, each of the ORs was multiplied by a weight, $\beta$, which allowed each of the genotypic/phenotypic variables to be given greater or lesser importance in the calculation of the total risk of breast cancer. Table 8 shows the weights obtained for each of the factors and for the age-breast density and age- age at menopause interactions. Table 8 also shows the statistical significance of the factors through the P-value represented in the last column. In the logistic regression, it was inferred if the probability of being a case was statistically related to each dependent variable. To test this dependence, a hypothesis test was performed, where the null hypothesis was that the variables were independent. The P-value determined the probability of making a mistake if the null hypothesis was rejected. The lower the P-value, the greater statistical significance in the relationship between variables and rejecting the null hypothesis means that there is a statistically significant relationship between both variables (alternative hypothesis).

The factors with the greatest statistical significance were found to be:

- Breast density
- Age at menopause
- PRS genotypic risk
- Age
- Age- age at menopause interaction
- Age-breast density interaction

Table 8. Results of logistic regression in the case-control study with the training cohort.

|  | Risk estimator | β | Standard error | Z-score | P>\|z\| |
|---|---|---|---|---|---|
| Breast density | -2.26343 | 0.103993176 | 0.57136 | -3.962 | $7.45 \cdot 10^{-5}$ *** |

(continued)

|  | Risk estimator | β | Standard error | Z-score | P>\|z\| |
|---|---|---|---|---|---|
| Age at first child | -0.74524 | 0.474620377 | 0.33319 | -2.237 | 0.02531*** |
| Biopsy | -1.80226 | 0.164925735 | 0.72945 | -2.471 | 0.01348 * |
| Age at menopause | -29.92687 | $1.00676 \cdot 10^{-13}$ | 2.85509 | -10.482 | $<2 \cdot 10^{-16}$ *** |
| Family history | -0.06459 | 0.93745174 | 0.18729 | -0.345 | 0.73021 |
| PRS genotypic risk | -1.33764 | 0.262464354 | 0.27437 | -4.875 | $1.09 \cdot 10^{-6}$ *** |
| Age | -0.76655 | 0.464613222 | 0.07628 | -10.049 | $<2 \cdot 10^{-16}$ *** |
| Age-breast density interaction | 0.03246 | 1.032992573 | 0.01031 | 3.148 | 0.00164 ** |
| Age- age at menopause interaction | 0.49873 | 1.646628724 | 0.05254 | 9.493 | $<2 \cdot 10^{-16}$ *** |
| Statistical significance codes: 0 (***), 0.001 (**), 0.01 (*), 0.05 (,), 0.1 ( ) | | | | | |

The weights correspond to the exponential of the risk estimator and are calculated using the following formula:

$$\beta = \exp{(risk\ estimator)}$$

Below is a table in which weights are ordered from highest to lowest impact in the calculation of the total risk of breast cancer.

Table 9. Factors ordered from highest to lowest weight in this example.

| Age-age at menopause interaction |
|---|
| Age-breast density interaction |
| Family history |
| Age at first child |
| Age |
| PRS genotypic risk |
| Biopsy |
| Breast density |
| Age at menopause |

Table 9 shows that the interactions of breast density and age at menopause, respectively, with age, are the factors with the greatest weight within the algorithm. Breast density and age at menopause are independently not as important for the risk calculation, but are important when analysed in interaction with age.

ROC curve

The performance of the global BRM interaction model was evaluated using receiver operating characteristic (ROC) curves. Figure 1 shows that the model that uses all the variables, both genotypic risk and the risk associated with all phenotypic factors, including interaction between terms, obtains better sensitivity and specificity values.

Z-score distribution between cases and controls

The Z-score ($z_i$) value is calculated using the following formula:

$$z_i = \frac{a_i - \mu}{s},$$

where

$$a_i = ln\left(\frac{P_{case}}{P_{control}}\right),$$

$$\mu = \frac{\sum_{i=1}^{N} a_i}{N},$$

$$s = \sqrt{\frac{\sum_{i=1}^{N} (a_i - \mu)^2}{N - 1}},$$

where $a_i$ is the risk estimator for women in the training cohort, i is between 1 and N, and N is the total number of women.

The distribution of the Z-score between cases and controls is shown in Figure 2.

Table 10 shows the High Risk data for different values of $\alpha$, where $\alpha$ is the type 1 error, the error of rejecting the null hypothesis, where the null hypothesis consists in belonging to the control/populational distribution and the alternative hypothesis consists in not belonging to the control/populational distribution, where if $\alpha$ = 0.1 the confidence level is 90%, if $\alpha$ = 0.2, the confidence level is 80% and if $\alpha$ = 0.3, the confidence level is 70%. Table 11 shows the Low-Risk data for different values of $\alpha$.

Table 10. High-Risk Data for different values of $\alpha$.

| $\alpha$ | 0.1 | 0.2 | 0.3 |
|---|---|---|---|
| Z-score | 1.28 | 0.84 | 0.5 |
| % high-risk cases | 18.614720 | 39.17749 | 64.93506 |
| % high-risk controls | 1.579779 | 8.21485 | 20.537125 |
| No. high-risk cases | 86 | 181 | 300 |
| No. high-risk controls | 10 | 52 | 130 |
| High-risk OR | 8.600000 | 3.480769 | 2.307692 |
| True positives (TP) | 86 | 181 | 300 |
| False negatives (FN) | 0 | 5 | 8 |
| Sensitivity | 1 | 0.9731183 | 0.974026 |

Table 11. Low-risk data for different values of $\alpha$.

| $\alpha$ | 0.3 | 0.2 | 0.1 |
|---|---|---|---|
| Z-score | -0.5 | -0.84 | -1.28 |
| % low-risk cases | 1.731602 | 1.082251 | 0 |
| % low-risk controls | 35.07109 | 26.85624 | 18.95735 |
| No. low-risk cases | 8 | 5 | 0 |
| No. low-risk controls | 222 | 170 | 120 |
| Low risk OR | 0.0159046 | 0.0086059 | 0 |
| True negatives (TN) | 222 | 170 | 120 |
| False positives (FP) | 130 | 52 | 10 |
| Sensitivity | 0.6306818 | 0.7657658 | 0.9230769 |

Distribution of the value of the OR between cases and controls

OR values were calculated using the following formula:

$$OR = \frac{BRM(X)}{BRM\ (median\ of\ the\ control\ population)}$$

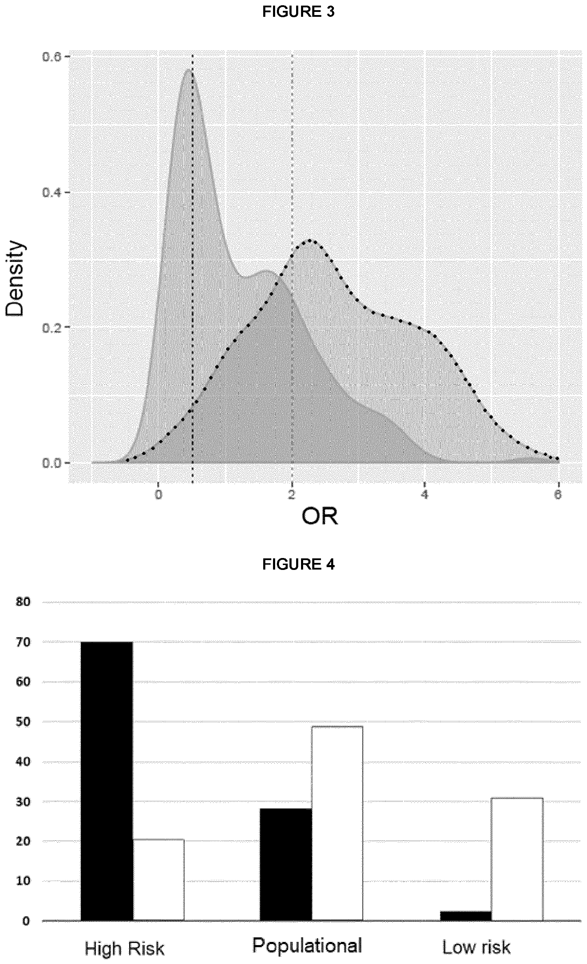

The distribution of OR values obtained for cases and controls is shown in Figure 3.

The following OR limit values were established:

- High Risk: OR $\geq$ 2
- Low risk: OR $\leq$ 0.5

In the high-risk category, there were 70% of cases and 20.5% of controls. In the populational-risk category, there were 27.74% of cases and 48.75% of controls. In the low-risk category, there were 2.26% of cases and 30.75% of controls. These percentages in each risk category are shown in Figure 4.

The following sensitivity and specificity values, positive predictive value and negative predictive value were extracted from the analysis of the data:

- Sensitivity: 97.4%
- Specificity: 60%
- Positive Predictive Value (PPV): 68.89%
- Negative Predictive Value (NPV): 99.32%

It is concluded that the BRM global interaction model, considering High Risk for OR $\geq$ 2 and Low Risk for OR $\leq$ 0.5, explained 70% of cases (high risk) and approximately 31% of controls (low risk).

For the calculation of the sensitivity and specificity of the test, the group of women who had been stratified in the populational risk group was not taken into account. Based on this, the calculation of sensitivity (ability to detect cases in the high-risk group) was performed taking into account only the group of women stratified by the BRM global interaction model as high risk (true positives vs false negatives).

The calculation of specificity (ability to detect controls in the low-risk group) was performed taking into account only the group of women stratified as low risk (true negatives vs. false positives).

**Example 2. Survival analysis with the proportional hazards model derived from the training cohort**

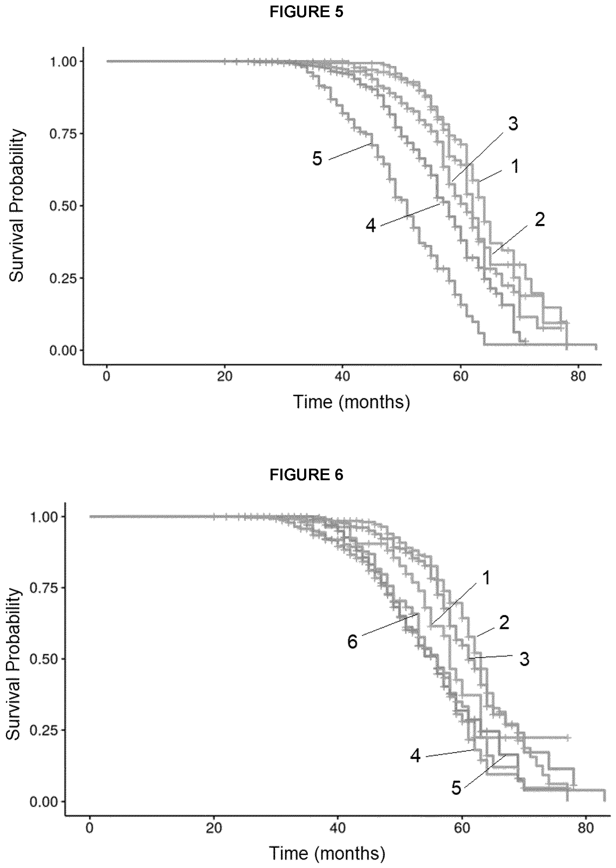

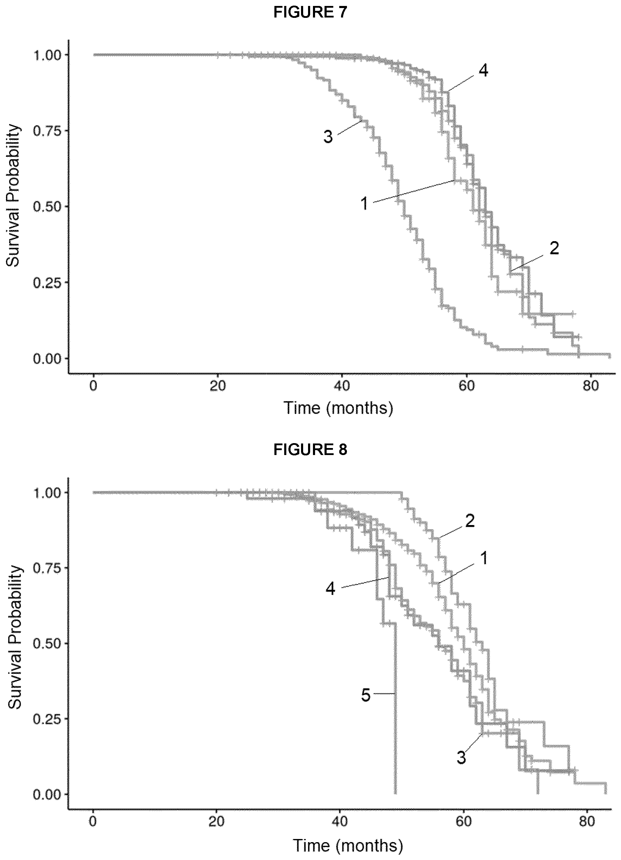

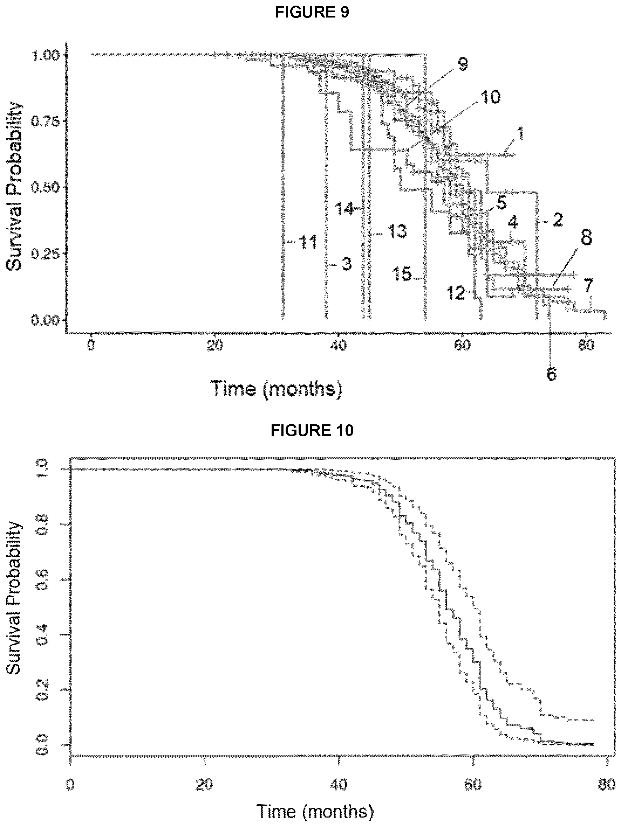

Individual survival analysis was carried out with the associated Cox proportional hazards model with the following factors: breast density (Figure 5), age at first child (Figure 6), age at menopause (Figure 7), family history (Figure 8) and PRS genotypic risk (Figure 9).

Survival analysis with the Cox proportional hazards model indicates that the risk was age-dependent and that the risk can be inferred using independent or explanatory variables.

The associated survival analysis of genotypic and phenotypic factors was performed individually and it was observed that all the factors used have statistical significance.

The Cox proportional hazards model was statistically significant with a P-value $\leq 10^{-16}$.

**Example 3. Prediction of the survival curve with the Cox proportional hazards model in a test group**

The survival analysis with the Cox proportional hazards model infers or predicts the survival or risk curve for each woman independently. Survival analysis with the Cox proportional hazards model allows to deduce the age intervals at which a high-risk woman will develop breast cancer.

In a group of high-risk women, called the test group, a prediction of the probability of survival was made using a Cox proportional hazards model using the following formula:

$$h(t) = h_o(t)exp\left(\sum_{i=1}^{m} \beta_i V_i\right)$$

where h(t) is the risk function of breast cancer, dependent on time t and risk factors $V_i$, $h_0(t)$ is the baseline hazard function, m is the number of risk factors, $V_i$ are the risk factors polygenic risk score (PRS), breast density, biopsy, age at menopause, age, family history, age at first child, age-breast density interaction and age- age at menopause interaction and $\beta_i$ corresponds to the coefficients described above. The baseline hazard function is the risk of a subject simply for his age and has a value of 0 for all risk factors.

Tables 12 and 13 show the results obtained with said survival analysis with the Cox proportional hazards model. The age column shows the age at which breast cancer was detected in women. The middle column shows the mean obtained with the survival analysis with the Cox proportional hazards model that represents the prediction of the age at which women would develop breast cancer according to the prediction model. The difference column shows the difference between the actual age at which breast cancer was detected in women and the prediction.

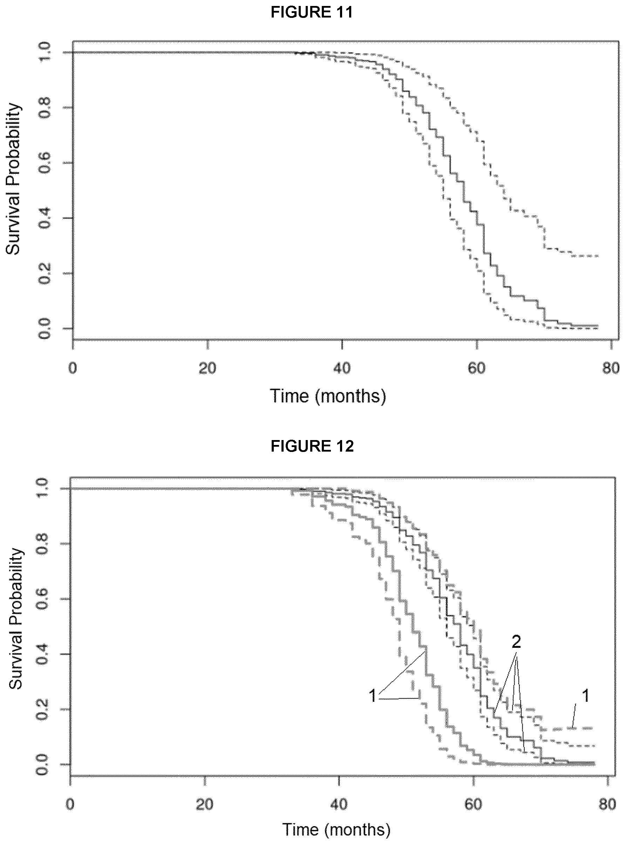

Figure 10 shows the survival curves inferred with the Cox proportional hazards model for one woman. Figure 11 shows the results for another woman. Figure 12 simultaneously shows the survival curves inferred with the Cox proportional hazards model for two different women.

Table 12. Survival prediction results with the Cox proportional hazards model in women in the test group.

| Woman | Lower confidence level = 0.95 | Upper confidence level = 0.95 | Age | Z-score | Mean | Difference |
|---|---|---|---|---|---|---|
| 2 | 51 | 55 | 53 | 1.3897472 | 53 | 0 |
| 4 | 49 | 58 | 47 | 2.2038663 | 52 | 5 |
| 6 | 55 | 60 | 39 | 1.4180718 | 56 | 17 |
| 8 | 50 | 58 | 51 | 2.0829441 | 53 | 2 |
| 19 | 51 | 60 | 48 | 1.7765090 | 53 | 5 |
| 23 | 55 | 64 | 63 | 1.1885624 | 58 | -5 |
| 24 | 49 | 60 | 36 | 1.7089641 | 51 | 15 |
| 28 | 60 | NA | 57 | 0.8466902 | 63 | 6 |
| 32 | 56 | 59 | 52 | 1.1552170 | 57 | 5 |
| 39 | 58 | 72 | 56 | 0.8417468 | 61 | 5 |
| 49 | 58 | 70 | 56 | 0.9495816 | 61 | 5 |
| 52 | 49 | 60 | 48 | 1.3298060 | 51 | 3 |
| 53 | 55 | 64 | 54 | 1.2080279 | 58 | 4 |
| 55 | 52 | 60 | 56 | 1.7048899 | 54 | -2 |

Table 13. Survival prediction results with the Cox proportional hazards model for women in the test group.

| Woman | Lower confidence level = 0.95 | Upper confidence level = 0.95 | Age | Type | Z-score | Mean | Difference |
|---|---|---|---|---|---|---|---|
| 40 | 56 | 60 | 54 | 2 | 1.0563884 | 58 | 4 |
| 158 | 55 | 58 | 51 | 2 | 1 .1190683 | 56 | 5 |
| 175 | 56 | 60 | 47 | 2 | 1.2120301 | 58 | 11 |
| 314 | 56 | 61 | 59 | 2 | 0.8994593 | 58 | -1 |
| 318 | 56 | 60 | 50 | 2 | 1.1294690 | 57 | 8 |
| 338 | 56 | 60 | 55 | 2 | 0.8702496 | 57 | 3 |
| 352 | 57 | 61 | 62 | 2 | 0.8769318 | 59 | -3 |
| 380 | 49 | 61 | 47 | 2 | 1.1634017 | 52 | 6 |
| 394 | 53 | 58 | 49 | 2 | 1.2035011 | 55 | 6 |
| 470 | 54 | 59 | 44 | 2 | 1.4630069 | 55 | 11 |

**REFERENCES**

Evans et al., Breast cancer risk assessment in 8,824 women attending a family history evaluation and screening programme. Fam Cancer., 2014, 13(2):189-96.

Bojesen et al., Multiple independent variants at the TERT locus are associated with telomere length and risks of breast and ovarian cancer. Nat Genet., 2013, 45(4):371-84, 384e 1-2.

Couch et al., Genome-wide association study in BRCA1 mutation carriers identifies novel loci associated with breast and ovarian cancer risk. PLoS Genet., 2013, 9(3):e1003212.

Gaudet et al., Identification of a BRCA2-specific modifier locus at 6p24 related to breast cancer risk. PLoS Genet., 2013, 9(3):e1003173.

Ghoussaini et al., Inherited genetic susceptibility to breast cancer: the beginning of the end or the end of the beginning? Am J Pathol., 2013, 183(4):1038-1051.

Michailidou et al., Large-scale genotyping identifies 41 new loci associated with breast cancer risk. Nat Genet., 2013, 45(4):353-61, 361e1-2.

Michailidou et al., Genome-wide association analysis of more than 120,000 individuals identifies 15 new susceptibility loci for breast cancer. Nat Genet., 2015, 47(4):373-80.

Michailidou et al., Association analysis identifies 65 new breast cancer risk loci. Nature, 2017, 551(7678):92-94.

Nickels et al., Evidence of gene-environment interactions between common breast cancer susceptibility loci and established environmental risk factors. PLoS Genet., 2013, 9(3):e1003284.

Osorio et al., DNA glycosylases involved in base excision repair may be associated with cancer risk in BRCA1 and BRCA2 mutation carriers. PLoS Genet., 2014, 10(4):e1004256.

Pharoah et al., Family history and the risk of breast cancer: a systematic review and meta-analysis. Int J Cancer, 1997, 71(5):800-9.

Pollán et al., Mammographic density and risk of breast cancer according to tumor characteristics and mode of detection: a Spanish population-based case-control study.

Breast Cancer Res, 2013, 15(1):R9. Shieh Yiwey et al. Breast cancer risk prediction using a clinical risk model and polygenic risk score. Breast Cancer Research and Treatment, 2016, 159: 513-525.

**Claims**

1. An *in vitro* method for determining the risk of developing breast cancer in a subject, wherein said method comprises combining the following parameters in a model:

    - the polygenic risk score (PRS) calculated from the risks associated with the presence of single nucleotide polymorphisms (SNPs) in a biological sample obtained from said subject,
    - the risk associated in said subject to the factors: breast density, biopsy, age at menopause, age, family history and age at first child and
    - the risk associated in said subject to the age-breast density and age-age at menopause interactions, wherein said interactions are interactions obtained by regression,
    wherein said risk of developing breast cancer in said subject is calculated from a model based on the combination of said parameters and wherein the result obtained in said model is indicative of said risk of developing breast cancer in said subject,
    wherein said single nucleotide polymorphisms (SNPs) are rs10069690, rs10088218, rs1045485, rs10472076, rs10474352, rs10771399, rs10816625, rs10931936, rs10941679, rs10965163, rs10995190, rs10995201, rs11196174, rs11196175, rs11199914, rs11249433, rs113577745, rs11552449, rs11571833, rs11814448, rs11820646, rs11977670, rs12422552, rs12443621, rs12710696, rs1292011, rs13039229, rs13066793, rs13281615, rs13294895, rs13329835, rs13365225, rs13387042, rs1353747, rs1432679, rs1466785, rs1550623, rs16857609, rs16886113, rs16991615, rs17356907, rs17631303, rs17817449, rs183211, rs184577, rs1895062, rs204247, rs2046210, rs2236007, rs2253407, rs2290203, rs2290854, rs2304277, rs2363956, rs2420946, rs2588809, rs2736108, rs2747652, rs27633, rs2823093, rs2943559, rs2981579, rs2992756, rs344008, rs3757322, rs3760982, rs3803662, rs3903072, rs4442975, rs45631563, rs4691139, rs4733664, rs4784227, rs4808801, rs4849887, rs4973768, rs554219, rs58058861, rs58847541, rs6001930, rs614367, rs616488, rs619373, rs6472903, rs6504950, rs6507583, rs6682208, rs67397200, rs6762644, rs6815814, rs6828523, rs704010, rs7072776, rs720475, rs72749841, rs72755295, rs72826962, rs7297051, rs73161324, rs7529522, rs75915166, rs78269692, rs8009944, rs8170, rs865686, rs889312, rs909116, rs9303542, rs9348512, rs9397437, rs941764, rs9693444, rs9790517, rs9790879, rs9833888, rs527616 and rs2380205,

wherein the combination, in said model, of the PRS score, the risk associated with the factors and the risk associated with the interactions, is carried out by the formula

$$BRM = \beta_0 + \beta_1 PRS + \beta_2 OR_{DM} + \beta_3 OR_{BIOP} + \beta_4 OR_{E1} + \beta_5 OR_{AF} + \beta_6 OR_{EM} + \beta_7 E + \beta_8 E + \beta_9 E$$

wherein $OR_{DM}$ is the value of the OR associated with breast density, $OR_{BIOP}$ is the value of the OR associated with the biopsy, $OR_{E1}$ is the value of the OR associated with the age at first child, $OR_{AF}$ is the value of the OR associated with the family history, $OR_{EM}$ is the value of the OR associated with the age at menopause, E is the age, $\beta_0$, $\beta_1$, $\beta_2$, $\beta_3$, $\beta_4$, $\beta_5$, $\beta_6$, $\beta_7$, $\beta_8$ and $\beta_9$ are coefficients determined in said model with a training cohort, $\beta_1$ is the coefficient associated with the PRS, $\beta_2$ is the coefficient associated with the breast density, $\beta_3$ is the coefficient associated with the biopsy, $\beta_4$ is the coefficient associated with the age at first child, $\beta_8$ is the coefficient associated with the family history, $\beta_6$ is the coefficient associated with the age at menopause, $\beta_7$ is the coefficient associated with the age, $\beta_8$ is the coefficient associated with the age-breast density interaction and $\beta_9$ is the coefficient associated with the age-age at menopause interaction,

wherein said model is a Cox proportional hazards model, wherein said risk of developing breast cancer in a subject is calculated by the formula

$$h(t) = h_o(t) exp\left(\sum_{i=1}^{m} \beta_i V_i\right)$$

where h(t) is the risk function of breast cancer, t is the time, $h_0(t)$ is the baseline hazard function, m is the number of risk factors, $V_i$ are the risk factors: polygenic risk score (PRS), breast density, biopsy, age at menopause, age, family history, age at first child, age-breast density interaction and age-age at menopause interaction; and $\beta_i$ corresponds to the coefficients $\beta_0$-$\beta_9$.

2. Method according to claim 1, wherein the PRS score is calculated by the formula

$$\prod_{k=1}^{n} OR_k^{x_k}$$

where each SNP is identified by a k value, where k is a value between 1 and n, where n is the total number of SNPs, $OR_k$ is the value of the OR (*odds ratio*) associated with SNP k, where $x_k$ is the number of alleles for SNP k and $x_k$ can have a value selected from the group consisting of 0, 1 and 2.

3. Method according to claim 1 or 2, wherein the coefficients $\beta_1$-$\beta_9$ have values between 0 and 2.

4. Method according to any one of claims 1-3, wherein said regression is logistic regression.

5. A method for stratifying or classifying subjects according to the risk of developing breast cancer which comprises determining the risk of developing breast cancer in said subjects according to the method of any of claims 1-4 and assigning or classifying subjects into low risk, populational risk and high risk groups.

6. Method according to claim 5, wherein a subject is assigned or classified:

- in the low risk group if the BRM value is equal to or greater than 0 and less than or equal to 0.5,
- in the populational risk group when the BRM value is greater than 0.5 and less than 2,
- in the high-risk group when the BRM value is equal to or greater than 2.

7. A method for selecting subjects at risk of developing breast cancer comprising determining the risk of developing breast cancer of said subjects according to the method of any of claims 1-4 and selecting subjects based on the value of said risk of developing breast cancer.

8. Method according to claim 7, wherein subjects assigned or classified into a high risk group are selected when the BRM value is equal to or greater than 2.

**Patentansprüche**

1. *In-vitro-Verfahren* zur Bestimmung des Risikos der Entwicklung von Brustkrebs bei einer Person, wobei das Verfahren das Kombinieren der folgenden Parameter in einem Modell umfasst:

- Der polygene Risikoscore (PRS), der aus den Risiken im Zusammenhang mit dem Vorhandensein von Einzelnukleotid-Polymorphismen (SNPs) in einer biologischen Probe, die von der Person erhalten wurde, berechnet wird,
- das Risiko, das bei der genannten Person mit den Faktoren: Brustdichte, Biopsie, Alter in den Wechseljahren, Alter, Familienanamnese und Alter beim ersten Kind verbunden ist, und
- das Risiko, das bei der Person mit der Dichte der Altersbrust und dem Alter bei Wechselwirkungen in den Wechseljahren verbunden ist, wobei die Wechselwirkungen solche sind, die durch Regression erlangt werden, wobei das Risiko der Entwicklung von Brustkrebs bei der Person, aus einem Modell berechnet wird, das auf der Kombination der Parameter basiert, und wobei das in dem Modell erhaltene Ergebnis indikativ für das Risiko der Entwicklung von Brustkrebs bei der Person ist,
wobei die Einzelnukleotid-Polymorphismen (SNPs) rs10069690, rs10088218, rs1045485, rs10472076, rs10474352, rs10771399, rs10816625, rs10931936, rs10941679, rs10965163, rs10995190, rs10995201, rs11196174, rs11196175, rs11199914, rs11249433, rs113577745, rs11552449, rs11571833, rs11814448, rs11820646, rs11977670, rs12422552, rs12443621, rs12710696, rs1292011, rs13039229, rs13066793, rs13281615, rs13294895, rs13329835, rs13365225, rs13387042, rs1353747, rs1432679, rs1466785, rs1550623, rs16857609, rs16886113, rs16991615, rs17356907, rs17631303, rs17817449, rs183211, rs184577, rs1895062, rs204247, rs2046210, rs2236007, rs2253407, rs2290203, rs2290854, rs2304277, rs2363956, rs2420946, rs2588809, rs2736108, rs2747652, rs27633, rs2823093, rs2943559, rs2981579, rs2992756, rs344008, rs3757322, rs3760982, rs3803662, rs3903072, rs4442975, rs45631563, rs4691139, rs4733664, rs4784227, rs4808801, rs4849887, rs4973768, rs554219, rs58058861, rs58847541, rs6001930, rs614367, rs616488, rs619373, rs6472903, rs6504950, rs6507583, rs6682208, rs67397200, rs6762644, rs6815814, rs6828523, rs704010, rs7072776, rs720475, rs72749841, rs72755295, rs72826962, rs7297051, rs73161324, rs7529522, rs75915166, rs78269692, rs8009944, rs8170, rs865686, rs889312, rs909116, rs9303542, rs9348512, rs9397437, rs941764, rs9693444, rs9790517, rs9790879, rs9833888, rs527616 und rs2380205 sind,
wobei die Kombination in dem Modell des PRS-Werts, des mit den Faktoren verbundenen Risikos und des mit den Wechselwirkungen verbundenen Risikos ausgeführt wird durch die Formel

$$BRM = \beta_0 + \beta_1 PRS + \beta_2 OR_{DM} + \beta_3 OR_{BIOP} + \beta_4 OR_{E1} + \beta_5 OR_{AF} + \beta_6 OR_{EM} + \beta_7 E + \beta_8 E + \beta_9 E$$

wobei $OR_{DM}$ der Wert des mit der Brustdichte assoziierten OR ist, $OR_{BIOP}$ der Wert des mit der Biopsie assoziierten OR ist, $OR_{E1}$ der Wert des mit dem Alter des ersten Kindes assoziierten OR ist, $OR_{AF}$ der Wert des mit der Familienanamnese assoziierten OR ist, $OR_{EM}$ der Wert des mit dem Alter in der Menopause assoziierten OR ist, E das Alter ist, $\beta_0$, $\beta_1$, $\beta_2$, $\beta_3$, $\beta_4$, $\beta_5$, $\beta_6$, $\beta_7$, $\beta_8$ und $\beta_9$ Koeffizienten sind, die in dem Modell mit einer Trainingskohorte bestimmt werden, $\beta_1$ ist der Koeffizient, der mit dem PRS assoziiert ist, $\beta_2$ ist der Koeffizient, der mit der Brustdichte assoziiert ist, $\beta_3$ ist der Koeffizient, der mit der Biopsie assoziiert ist, $\beta_4$ ist der Koeffizient, der mit dem Alter des ersten Kindes assoziiert ist, $\beta_5$ ist der Koeffizient, der mit der Familienanamnese assoziiert ist, $\beta_6$ ist der Koeffizient, der mit dem Alter in der Menopause assoziiert ist, $\beta_7$ ist der Koeffizient, der mit dem Alter assoziiert ist, $\beta_8$ ist der Koeffizient, der mit der Dichte-Wechselwirkung zwischen Alter und Brust assoziiert ist und $\beta_9$ ist der Koeffizient, der mit dem Alter bei Wechselwirkung zwischen Alter und Brust in den Wechseljahren assoziiert ist,
wobei das Modell ein Coxsches Regressionsmodell ist, wobei das Risiko der Entwicklung von Brustkrebs bei einer Person, berechnet wird durch die Formel

$$h(t) = h_o(t) exp \left( \sum_{i=1}^{m} \beta_i V_i \right)$$

wobei h(t) die Risikofunktion von Brustkrebs ist, t die Zeit ist, $h_0(t)$ die Basislinien-Risikofunktion ist, m die Anzahl der Risikofaktoren ist, $V_i$ sind die Risikofaktoren: polygener Risikoscore (PRS), Brustdichte, Biopsie, Alter in der Menopause, Alter, Familiengeschichte, Alter beim ersten Kind, Alter-Brust-Dichte-Interaktion und Alter-Alter in der Menopause-Interaktion; und $\beta_i$ den Koeffizienten $\beta_0$-$\beta_9$ entspricht.

2. Verfahren nach Anspruch 1, wobei der PRS-Wert berechnet wird durch die Formel

$$\prod_{k=1}^{n} OR_k^{x_k}$$

wobei jeder SNP durch einen k-Wert identifiziert wird, wobei k ein Wert zwischen 1 und n ist, wobei n die Gesamtzahl der SNPs ist, $OR_k$ der Wert des mit SNP k verbundenen OR *(Odds Ratio)* ist, wobei $X_k$ die Anzahl der Allele für SNP k ist und $X_k$ einen Wert haben kann, ausgewählt aus der Gruppe bestehend aus 0, 1 und 2.

3. Verfahren nach Anspruch 1 oder 2, wobei die Koeffizienten $\beta_1$-$\beta_9$ Werte zwischen 0 und 2 aufweisen.

4. Verfahren nach einem der Ansprüche 1-3, wobei die Regression eine logistische Regression ist.

5. Verfahren zum Stratifizieren oder Klassifizieren von Personen gemäß dem Risiko der Entwicklung von Brustkrebs, welches das Bestimmen des Risikos der Entwicklung von Brustkrebs bei diesen Personen umfasst, gemäß dem Verfahren nach einem der Ansprüche 1-4, und das Zuordnen oder Klassifizieren von Personen in Gruppen mit geringem Risiko, Bevölkerungsrisiko und hohem Risiko.

6. Verfahren nach Anspruch 5, wobei eine Person zugeordnet oder klassifiziert wird:

- in der Gruppe mit geringem Risiko, wenn der BRM-Wert gleich oder größer als 0 und kleiner oder gleich 0,5 ist,
- in der Bevölkerungsrisikogruppe, wenn der BRM-Wert größer als 0,5 und kleiner als 2 ist,
- in der Hochrisikogruppe, wenn der BRM-Wert gleich oder größer als 2 ist.

7. Verfahren zum Auswählen von Personen, bei denen das Risiko der Entwicklung von Brustkrebs besteht, umfassend das Bestimmen des Risikos der Entwicklung von Brustkrebs bei diesen Personen, gemäß dem Verfahren nach einem der Ansprüche 1-4 und das Auswählen von Personen, basierend auf dem Wert des Risikos der Entwicklung von Brustkrebs.

8. Verfahren nach Anspruch 7, wobei Personen, die in eine Hochrisikogruppe eingeteilt oder klassifiziert sind, ausgewählt werden, wenn der BRM-Wert gleich oder größer als 2 ist.

**Revendications**

1. Procédé *in vitro* pour déterminer le risque de développer un cancer du sein chez un sujet, dans lequel ledit procédé comprend la combinaison des paramètres suivants dans un modèle :

- le score de risque polygénique (PRS) calculé à partir des risques associés à la présence de polymorphismes mononucléotidiques (SNP) dans un échantillon biologique prélevé sur ledit sujet,
- le risque associé chez ledit sujet aux facteurs : densité mammaire, biopsie, âge à la ménopause, âge, antécédents familiaux et âge au premier enfant et
- le risque associé chez ledit sujet aux interactions âge-densité des seins et âge-âge lors de la ménopause, lesdites interactions étant des interactions obtenues par régression,

dans lequel ledit risque de développer un cancer du sein chez ledit sujet est calculé à partir d'un modèle basé sur la combinaison desdits paramètres et dans lequel le résultat obtenu dans ledit modèle est indicatif dudit risque de développer un cancer du sein chez ledit sujet,

dans lequel lesdits polymorphismes mononucléotidiques (SNP) sont rs10069690, rs10088218, rs1045485, rs10472076, rs10474352, rs10771399, rs10816625, rs10931936, rs10941679, rs10965163, rs10995190, rs10995201, rs11196174, rs11196175, rs11199914, rs11249433, rs113577745, rs11552449, rs11571833, rs11814448, rs11820646, rs11977670, rs12422552, rs12443621, rs12710696, rs1292011, rs13039229, rs13066793, rs13281615, rs13294895, rs13329835, rs13365225, rs13387042, rs1353747, rs1432679, rs1466785, rs1550623, rs16857609, rs16886113, rs16991615, rs17356907, rs17631303, rs17817449, rs183211, rs184577, rs1895062, rs204247, rs2046210, rs2236007, rs2253407, rs2290203, rs2290854, rs2304277, rs2363956, rs2420946, rs2588809, rs2736108, rs2747652, rs27633, rs2823093, rs2943559, rs2981579, rs2992756, rs344008, rs3757322, rs3760982, rs3803662, rs3903072, rs4442975, rs45631563, rs4691139, rs4733664, rs4784227, rs4808801, rs4849887, rs4973768, rs554219, rs58058861, rs58847541, rs6001930, rs614367, rs616488, rs619373, rs6472903, rs6504950, rs6507583, rs6682208, rs67397200, rs6762644, rs6815814, rs6828523, rs704010, rs7072776, rs720475, rs72749841, rs72755295, rs72826962, rs7297051, rs73161324, rs7529522, rs75915166, rs78269692, rs8009944, rs8170, rs865686, rs889312, rs909116, rs9303542, rs9348512, rs9397437, rs941764, rs9693444, rs9790517, rs9790879, rs9833888, rs527616 et rs2380205,

dans lequel la combinaison, dans ledit modèle, du score PRS, du risque associé aux facteurs et du risque associé aux interactions, est réalisée par la formule

$$BRM = \beta_0 + \beta_1 PRS + \beta_2 OR_{DM} + \beta_3 OR_{BIOP} + \beta_4 OR_{E1} + \beta_5 OR_{AF} + \beta_6 OR_{EM} + \beta_7 E + \beta_8 E + \beta_9 E$$

où $OR_{DM}$ est la valeur de l'OR associée à la densité mammaire, $OR_{BIOP}$ est la valeur de l'OR associée à la biopsie, $OR_{E1}$ est la valeur de l'OR associée à l'âge au premier enfant, $OR_{AF}$ est la valeur de l'OR associée aux antécédents familiaux, $OR_{EM}$ est la valeur de l'OR associée à l'âge à la ménopause, E est l'âge, $\beta_0$, $\beta_1$, $\beta_2$, $\beta_3$, $\beta_4$, $\beta$s, $\beta$s, $\beta_7$, $\beta_8$ et $\beta_9$ sont des coefficients déterminés dans ledit modèle avec une cohorte d'entraînement, $\beta_l$ est le coefficient associé au PRS, $\beta$2 est le coefficient associé à la densité mammaire, $\beta_3$ est le coefficient associé à la biopsie, $\beta_4$ est le coefficient associé à l'âge au premier enfant, $\beta_5$ est le coefficient associé aux antécédents familiaux, $\beta_6$ est le coefficient associé à l'âge à la ménopause, $\beta_7$ est le coefficient associé à l'âge, $\beta_8$ est le coefficient associé à l'interaction âge-densité mammaire et $\beta_9$ est le coefficient associé à l'âge-âge à l'interaction ménopausique,

dans lequel ledit modèle est un modèle de risques proportionnels de Cox, dans lequel ledit risque de développer un cancer du sein chez un sujet est calculé par la formule

$$h(t) = h_o(t) exp\left(\sum_{i=1}^{m} \beta_i V_i\right)$$

où h(t) est la fonction de risque du cancer du sein, t est le temps, $h_0(t)$ est la fonction de risque de base, m est le nombre de facteurs de risque, $V_i$ sont les facteurs de risque : score de risque polygénique (PRS), densité mammaire, biopsie, âge à la ménopause, âge, antécédents familiaux, âge au premier enfant, interaction âge-densité mammaire et âge-âge à l'interaction ménopausique ; et $\beta_i$ correspond aux coefficients $\beta_0$-$\beta_9$.

2. Procédé selon la revendication 1, dans lequel le score PRS est calculé par la formule

$$\prod_{k=1}^{n} OR_k^{x_k}$$

où chaque SNP est identifié par une valeur k, où k est une valeur comprise entre 1 et n, où n est le nombre total de SNPs, ORk est la valeur du OR *(ratio d'odds)* associé à SNP k, où $x_k$ est le nombre d'allèles pour SNP k et $x_K$

peut avoir une valeur choisie dans le groupe constitué de 0, 1 et 2.

3. Procédé selon la revendication 1 ou 2, dans lequel les coefficients $\beta_1$-$\beta_9$ ont des valeurs comprises entre 0 et 2.

4. Procédé selon l'une quelconque des revendications 1 à 3, dans lequel ladite régression est une régression logistique.

5. Procédé pour stratifier ou classer des sujets selon le risque de développer un cancer du sein, qui comprend la détermination du risque de développer un cancer du sein chez lesdits sujets selon le procédé de l'une quelconque des revendications 1 à 4 et l'affectation ou la classification de sujets dans des groupes à faible risque, à risque de population et à risque élevé.

6. Méthode selon la revendication 5, dans lequel un sujet est assigné ou classifié :

   - dans le groupe à faible risque si la valeur BRM est égale ou supérieure à 0 et inférieure ou égale à 0,5,
   - dans le groupe de risque de population lorsque la valeur BRM est supérieure à 0,5 et inférieure à 2,
   - dans le groupe à haut risque lorsque la valeur BRM est égale ou supérieure à 2.

7. Procédé de sélection de sujets à risque de développer un cancer du sein comprenant la détermination du risque de développer un cancer du sein desdits sujets selon le procédé de l'une quelconque des revendications 1 à 4 et la sélection de sujets sur la base de la valeur dudit risque de développer un cancer du sein.

8. Procédé selon la revendication 7, dans lequel des sujets affectés ou classés dans un groupe à haut risque sont sélectionnés lorsque la valeur de BRM est égale ou supérieure à 2.

FIGURE 1

FIGURE 2

FIGURE 3

FIGURE 4

FIGURE 5

FIGURE 6

## FIGURE 7

## FIGURE 8

FIGURE 9

FIGURE 10

## FIGURE 11

## FIGURE 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2438193 A1

**Non-patent literature cited in the description**

- **EVANS et al.** Breast cancer risk assessment in 8,824 women attending a family history evaluation and screening programme. *Fam Cancer.,* 2014, vol. 13 (2), 189-96
- **BOJESEN et al.** Multiple independent variants at the TERT locus are associated with telomere length and risks of breast and ovarian cancer. *Nat Genet.,* 2013, vol. 45 (4), 371-84, 384e 1-2
- **COUCH et al.** Genome-wide association study in BRCA1 mutation carriers identifies novel loci associated with breast and ovarian cancer risk. *PLoS Genet.,* 2013, vol. 9 (3), e1003212
- **GAUDET et al.** Identification of a BRCA2-specific modifier locus at 6p24 related to breast cancer risk. *PLoS Genet.,* 2013, vol. 9 (3), e1003173
- **GHOUSSAINI et al.** Inherited genetic susceptibility to breast cancer: the beginning of the end or the end of the beginning?. *Am J Pathol.,* 2013, vol. 183 (4), 1038-1051
- **MICHAILIDOU et al.** Large-scale genotyping identifies 41 new loci associated with breast cancer risk. *Nat Genet.,* 2013, vol. 45 (4), 353-61, 361e1-2
- **MICHAILIDOU et al.** Genome-wide association analysis of more than 120,000 individuals identifies 15 new susceptibility loci for breast cancer. *Nat Genet.,* 2015, vol. 47 (4), 373-80
- **MICHAILIDOU et al.** Association analysis identifies 65 new breast cancer risk loci. *Nature,* 2017, vol. 551 (7678), 92-94
- **NICKELS et al.** Evidence of gene-environment interactions between common breast cancer susceptibility loci and established environmental risk factors. *PLoS Genet.,* 2013, vol. 9 (3), e1003284
- **OSORIO et al.** DNA glycosylases involved in base excision repair may be associated with cancer risk in BRCA1 and BRCA2 mutation carriers. *PLoS Genet.,* 2014, vol. 10 (4), e1004256
- **PHAROAH et al.** Family history and the risk of breast cancer: a systematic review and meta-analysis. *Int J Cancer,* 1997, vol. 71 (5), 800-9
- **POLLÁN et al.** *Mammographic density and risk of breast cancer according to tumor characteristics and mode of detection: a Spanish population-based case-control study*
- *Breast Cancer Res,* 2013, vol. 15 (1), R9
- **SHIEH YIWEY et al.** Breast cancer risk prediction using a clinical risk model and polygenic risk score. *Breast Cancer Research and Treatment,* 2016, vol. 159, 513-525